# FASCICULE DE BREVET EUROPEEN

(11) **EP 0 483 023 B1**
(45) Date de publication et mention de la délivrance du brevet: **09.08.1995**
(21) Numéro de dépôt: 91420367.4
(22) Date de dépôt: 18.10.1991
(51) Int. Cl.: A61F 2/34, A61F 2/32

(54) **Implant cotyloidien**
Hüftgelenkpfanne
Acetabular cup

(30) Priorité: 22.10.1990 FR 9013327
(43) Date de publication de la demande: 29.04.1992
(73) Titulaire: ICP FRANCE, Société Anonyme, F-52003 Chaumont Cédex (FR)
(72) Inventeur: Landanger, Joel, F-52330 Colombey-Les-Deux-Eglises (FR); Braud, Gérard, F-01000 Bourg en Bresse (FR); Schiffrine, Patrick, F-74290 Veyrier du Lac (FR); Bost, Joel, F-69480 Anse (FR); Machet, Pierre, F-74700 Sallanches (FR); Revel, Jean-Jacques, La Réunion (FR)
(74) Mandataire: Dupuis, François

(56) Documents cités:
- EP-A- 0 353 171
- FR-A- 2 598 908
- FR-A- 2 649 005
- FR-A- 2 651 675
- US-A- 2 910 978

## Description

D'une manière parfaitement connue, ce type d'implant est destiné à remplacer la cavité cotyloïde de l'os illiaque dans laquelle s'articule la tête du fémur. Généralement, l'anneau est réalisé en métal tandis que le noyau est exécuté en polyéthylène.

Concernant la fixation de l'anneau dans la cavité cotyloïde, cette dernière peut s'effectuer de différentes façons. L'invention concerne plus particulièrement, les anneaux impactés au moyen de vis.

On peut citer par exemple l'enseignement du brevet FR 2598908, qui enseigne une prothèse cotyloïdienne à impactage vissage, sans ciment dans le cotyle. Cette prothèse comprend un anneau présentant une large échancrure formée d'un côté depuis son bord périphérique jusqu'à sensiblement sa zone polaire. En outre, l'anneau présente des aspérités aptes à s'implantées dans le cotyle osseux.

Un des problèmes que se propose de résoudre l'invention, est de faciliter et d'améliorer l'impaction et la fixation de l'anneau dans la cavité cotyloïde.

Pour résoudre le problème posé d'assurer une stabilité primaire facilitant la mise en place des vis, et pour assurer une pose facile et rapide par un matériel ancillaire en conservant une bonne visibilité du fond de la cavité cotyloïde et pour donner une certaine élasticité à l'anneau, en vue de faciliter son impaction, il a été conçu et mis au point un implant du type de ceux comprenant un anneau qui présente une large échancrure formée d'un côté, depuis son bord périphérique jusqu'à la zone polaire, le bord périphérique présentant à proximité de ladite échancrure et d'une manière symétrique, des aspérités aptes à s'implanter dans les cornes du cotyle osseux pour assurer une stabilité primaire, remarquable en ce que la cavité interne de l'anneau est excentrée par rapport à la cavité externe

Avantageusement, l'échancrure a une forme de serrure en présentant une partie circulaire au niveau de la zone polaire, ladite partie se raccordant d'une manière symétrique par de larges rayons de courbure au bord périphérique, en constituant une lumière étranglée.

Il apparait donc possible de contrôler le positionnement de l'anneau au fond du cotyle afin de s'assurer du bon contact entre ledit anneau et l'os souschondral.

Un autre problème que se propose de résoudre l'invention est de pouvoir, à volonté, orienter angulairement les vis d'impaction pour diriger ces dernières dans les parties souhaitées du cotyle, en fonction de l'état de ce dernier.

Ce problème est résolu en ce que la tête des vis d'impaction est de forme hémisphérique en étant logée dans des trous de formes complémentaires formés dans la partie de plus grande épaisseur de l'anneau.

Pour résoudre le problème posé de la réhabilitation osseuse, la périphérie externe de l'anneau est revêtue d'une couche de titane poreux, sous forme de plasma spray.

Pour résoudre le problème posé d'éviter tout risque de rotation du noyau dans l'anneau, ce dernier présente en débordement de son bord périphérique interne, des ergots inclinés aptes à coopérer avec le noyau.

Avantageusement, le noyau est conformé pour être clipsé à l'intérieur de l'anneau.

Suivant une autre caractéristique, l'anneau, tel que défini, peut recevoir des noyaux standards ou bien des noyaux présentant un débord latéral. Dans ce dernier cas, l'orientation du noyau est déterminée avant son encliquetage dans un noyau.

L'invention est exposée ci-après plus en détail, à l'aide des dessins annexés, dans lesquels :
La figure 1 est une vue en plan de l'anneau.
La figure 2 est une vue en coupe considérée selon la ligne 2-2 de la figure 1.
La figure 3 est une vue partielle et en coupe à grande échelle, montrant les agencements du noyau pour le logement des vis d'impaction.
La figure 4 est une vue de face d'une forme de réalisation du noyau.
La figure 5 montre par une vue de face, une autre forme de réalisation du noyau.

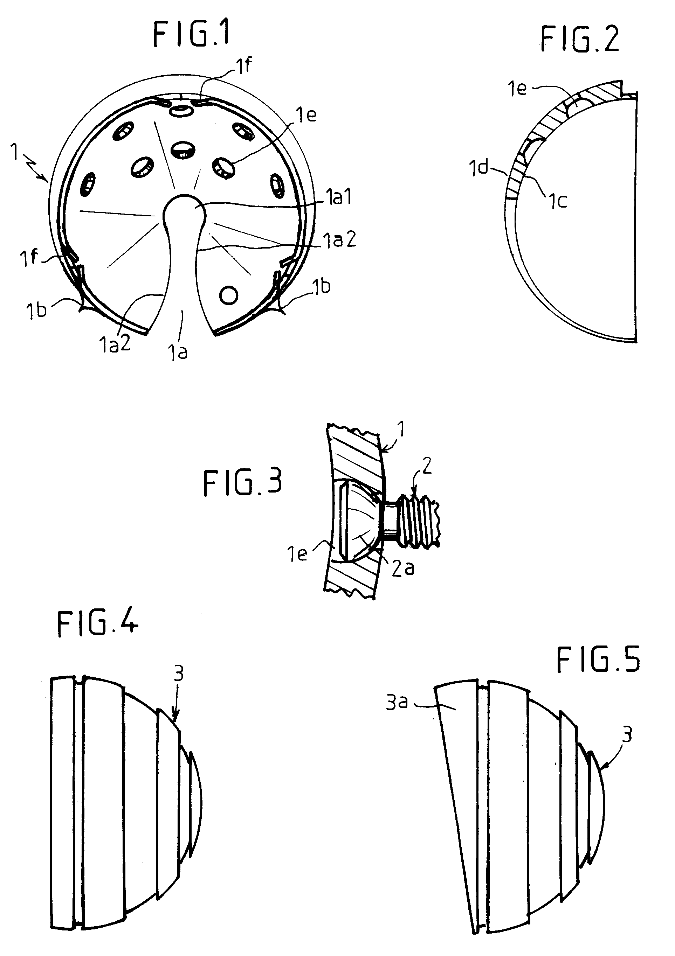

Comme le montre la figure 1, l'anneau, désigné dans son ensemble par (1), présente sur un côté, une large échancrure (1a) formée depuis le bord périphérique de l'anneau jusqu'au niveau de sa zone polaire. Cette échancrure (1a) est profilée sous forme d'un trou de serrure afin de conserver une bonne visibilité au cours d'une intervention. Dans ce but, l'échancrure (1a) présente au niveau de la zone polaire, une partie circulaire (1a1) qui se raccorde, d'une manière symétrique, au bord périphérique supérieur de l'anneau, par de larges rayons de courbures (1a2), de manière à constituer une lumière étranglée.

A noter également que l'échancrure profilée (1a) donne une certaine élasticité à l'ensemble de l'anneau favorisant ainsi son impaction dans la cavité cotyloïde. En outre, cette élasticité peut être encore améliorée par le fait que la cavité interne (1c) de l'anneau est excentrée par rapport à la cavité externe (1d) de l'anneau.

Suivant une autre caractéristique, le bord périphérique de l'anneau présente, notamment du côté de l'échancrure (1a), des aspérités d'ancrage (1b) aptes à coopérer dans les cornes du cotyle osseux. Ces aspérités (1b) apparaissent en débordement de la périphérie externe de l'anneau et forment des ailerons profilés notamment triangulaires.

Avantageusement, ces ailerons d'ancrage (1b) sont disposés symétriquement par rapport à l'échancrure (1a). La fonction de ces ailerons est d'assurer une stabilité primaire de l'anneau pour permettre la pose facile et rapide des vis d'impaction (2).

La fixation de l'anneau s'effectue au moyen des vis autotaraudeuses (2) réalisées, par exemple, d'une manière connue, en alliage de titane tel que le TA6V4.

La tête (2a) des vis (2) est de forme hémisphérique en étant logée dans des trous de formes complémentaires (1e) formés dans la partie de plus grande épaisseur de l'anneau. La sphéricité des têtes de vis autorise un débattement de ces dernières selon un angle d'enrivon 30°. A noter que les trous (1e) sont disposés dans la partie de plus grande épaisseur de l'anneau selon deux rangées parallèles.

La périphérie externe (1d) de l'anneau est revêtue d'une couche de titane poreux sous forme de plasma spray assurant, de manière connue, la réhabilitation osseuse d'une manière progressive.

Suivant une autre caractéristique, et comme le montre notamment la figure 1, l'anneau présente en débordement de son bord périphérique interne, des ergots inclinés (1f) aptes à coopérer avec le noyau (3). Ces ergots assurent le blocage en rotation du noyau (3).

En ce qui concerne le noyau (3), réalisé de manière connue en polyéthylène, ce dernier est profilé et agencé pour être clipsé dans la cavité interne du noyau. En fonction du cas pathologique à traiter, le noyau (3) peut être du type standard (figure 4), ou bien présenté un débord latéral (3a) (figure 5). Ce débord (3a), qui forme un angle par rapport au plan horizontal de l'ordre de 10°, limite le risque de luxation.

Les avantages ressortent bien de la description, en particulier on souligne et on rappelle :
- une stabilité primaire facilitant la mise en place des vis d'impaction,
- une bonne visibilité pour la mise en place facile et rapide de l'anneau par le matériel ancillaire,
- un revêtement plasma spray permettant la réhabilitation osseuse,
- l'utilisation de différents types de noyaux, dont certains présentent un débord limitant les risques de luxation.

## Revendications

1. Implant cotyloïdien comprenant des vis d'impaction (2), un anneau (1) impacté avec lesdites vis (2) et un noyau (3), ledit anneau (1) présentant une large échancrure (1a) formée d'un côté depuis son bord périphérique jusqu'à la zone polaire, le bord périphérique présentant, à proximité de ladite échancrure et de manière symétrique, des aspérités (1b) aptes à s'implanter dans les cornes du cotyle osseux pour assurer une stabilité primaire, caractérise en ce que la cavité interne (1c) de l'anneau est excentrée par rapport à la cavité externe (1d).

2. Implant selon la revendication 1, caractérisé en ce que l'échancrure (1a) présente, au niveau de la zone polaire, une partie circulaire (1a1), se raccordant de manière symétrique, par de larges rayons de courbure (1a2), au bord périphérique situé du côté de la zône amincie de l'anneau, en constituant une lumière étranglée.

3. Implant selon la revendication 1, caractérisé en ce que la tête (2a) des vis d'impaction (2) est de forme hémisphérique en étant logée dans des trous (1e) de formes complémentaires formées dans la partie de plus grande épaisseur de l'anneau.

4. Implant selon la revendication 1, caractérisé en ce que la périphérie externe (1d) de l'anneau est revêtue d'une couche de titane poreux, sous forme de plasma spray.

5. Implant selon la revendication 1, caractérisé en ce que l'anneau (1) présente en débordement de son bord périphérique interne, des ergots inclinés (1f) aptes à coopérer avec le noyau (3).

6. Implant selon l'une quelconque des revendications 1 et 5, caractérisé en ce que le noyau (3) est conformé pour être clipsé à l'intérieur de l'anneau (1).

7. Implant selon la revendication 6, caractérisé en ce que le noyau (3) présente un débord latéral (3a).

## Claims

1. Cotyloidal implant comprising impaction screws (2), a ring (1) impacted with said screws (2) and a core (3), said ring (1) having a large cutout (1a) on one side from its peripheral edge to the polar area, the peripheral edge having, close to said cutout and symmetrically located, protrusions (1b) capable of fitting into the cusps of the bony acetabulum in order to ensure primary stability, characterised in that the internal cavity (1c) of the ring is offcentred with respect to the external cavity (1d).

2. Implant as claimed in claim 1 characterised in that the cutout (1a) has, near the polar area, a circular part (1a1) symmetrically connected, by wide radii of curvature (1a2), to the peripheral edge situated on the side of the thinner area of the ring constituting a restricted aperture.

3. Implant as claimed in claim 1 characterised in that the head (2a) of the impaction screws (2) is of hemispherical shape and is accommodated in holes (1e) of matching shapes formed in the thickest part of the ring.

4. Implant as claimed in claim 1 characterised in that the outer periphery (1d) of the ring is covered in a coat of porous titanium in the form of a plasma spray.

5. Implant as claimed in claim 1 characterised in that ring (1) has slanting lugs (1f) projecting beyond its peripheral edge suitable for cooperating with core (3).

6. Implant as claimed in any of claims 1 and 5 characterised in that core (3) is shaped so that it clips into ring (1).

7. Implant as claimed in claim 6 characterised in that core (3) has a lateral rim (3a).

## Patentansprüche

1. Azetabulumimplantat bestehend aus Anschlagschrauben (2), einem mit besagten Schrauben (2) angeschlagenen Ring (1) und einem Kern (3), wobei der Ring (1) einen breiten bogenförmigen Ausschnitt (1a) aufweist, der auf einer Seite vom äußeren Rand bis zum Polbereich reicht, während der Außenrand in der Nähe dieses Ausschnitts symmetrisch angeordnete Unebenheiten (1b) aufweist, die geeignet sind, sich in den Hörnern der Knochenpfanne festzusetzen, um eine primäre Stabilität zu gewährleisten, dadurch gekennzeichnet, daß die innere Aushöhlung (1c) des Rings gegenüber der äußeren Aushöhlung (1d) exzentrisch versetzt ist.

2. Implantat nach Anspruch 1, dadurch gekennzeichnet, daß der bogenförmige Ausschnitt (1a) im Polbereich einen kreisförmigen Teil (1a1) aufweist, der symmetrisch durch breite Krümmungsradien (1a2) mit dem Außenrand auf der Seite des verjüngten Ringbereichs in Verbindung steht und dabei einen verschmälerten Schlitz bildet.

3. Implantat nach Anspruch 1, dadurch gekennzeichnet, daß der Kopf (2a) der Anschlagschrauben (2) halbkugelförmig ausgebildet ist und in komplementär ausgeformten Löchern im dickeren Teil des Rings sitzt.

4. Implantat nach Anspruch 1, dadurch gekennzeichnet, daß der Außenrand (1d) des Rings mit einer porösen Titanschicht in Form von Plasmaspray überzogen ist.

5. Implantat nach Anspruch 1, dadurch gekennzeichnet, daß der Ring (1) über den inneren Außenrand schräg hinausragende Nasen (1f) aufweist, die geeignet sind, mit dem Kern (3) zusammenzuwirken.

6. Implantat nach irgendeinem der Ansprüche 1 und 5, dadurch gekennzeichnet, daß der Kern (3) so ausgebildet ist, um in den Ring (1) einzuschnappen.

7. Implantat nach Anspruch 6, dadurch gekennzeichnet, daß der Kern (3) einen seitlichen Vorsprung (3a) aufweist.
